# EUROPEAN PATENT APPLICATION

(11) **EP 3 741 425 A1**
(43) Date of publication of application: **25.11.2020**
(21) Application number: 19176409.1
(22) Date of filing: 24.05.2019
(51) Int. Cl.: A61N 1/05, A61N 1/368

(54) **A LEAD FOR A PACEMAKER DEVICE CONFIGURED TO PROVIDE FOR AN INTRA-CARDIAC PACING AT THE HIS BUNDLE**

(71) Applicant: BIOTRONIK SE & Co. KG, 12359 Berlin (DE)
(72) Inventor: Dörr, Thomas, 12437 Berlin (DE); Lang, Volker, 12161 Berlin (DE); Swenson, Kurt, Dayton, OR 97114 (US)
(74) Representative: Keck, Hans-Georg

(57) **Abstract**

A lead (10, 11) for a pacemaker device (1) configured to provide for an intra-cardiac pacing at the HIS bundle (H) comprises a shaft (105) having a first end (100) to be connected to a stimulation device (12) of the pacemaker device (1), and a second end (101) opposite to the first end (100), wherein the second end (101) is to be implanted into intra-cardiac tissue (M) in the vicinity of the HIS bundle (H) of a patient's heart. An electrode arrangement (104) is arranged on the shaft (105) in proximity to the second end (101), the electrode arrangement (104) comprising a multiplicity of electrodes (104A-D) to engage with intra-cardiac tissue (M) in the vicinity of the HIS bundle (H). In this way a lead for a pacemaker device is provided which in an easy and effective manner allows for a pacing at the HIS bundle.

## Description

### TECHNICAL FIELD

The instant invention generally relates to a lead for a pacemaker device configured to provide for an intra-cardiac pacing at the HIS bundle and to a pacemaker device.

### BACKGROUND

A lead of this kind comprises a shaft having a first end to be connected to a stimulation device of the pacemaker device, and a second end opposite to the first end. The second end is to be implanted into intra-cardiac tissue in the vicinity of the HIS bundle of a patient's heart in order to provide for a pacing at the HIS bundle by injecting suitable stimulation signals into intra-cardiac tissue at the HIS bundle.

Approaches exist to provide for a so-called HIS bundle pacing in order to synchronously pace both the right ventricle and the left big ventricle by injecting a stimulus via the HIS bundle. Within a pacemaker device using leads to inject a stimulus a stimulation device is subcutaneously implanted into a patient at a location removed from the patient's heart, one or multiple leads extending from the stimulation device for example through the superior vena towards the patient's heart to access for example the right atrium.

In particular, for implanting a lead at the HIS bundle, the lead may be directed into collagen tissue in a layer between the atria and the ventricles. The lead herein, with its second, distal end, may be inserted into the collagen tissue from the right atrium from above the annular ring of the tricuspid valve or may be guided past the annulus to engage with the collagen tissue from the right ventricle.

For achieving an effective pacing at the HIS bundle, proper positioning of the lead at the HIS bundle is required. The lead has to extended towards the HIS bundle such that a stimulation of the HIS bundle by an injected stimulus is achieved. Herein, it is a desire that not too few, but also not too many cells at the HIS bundle are stimulated. If too few cells are stimulated, potentially only one of the right bundle branch and the left bundle branch of the HIS bundle are stimulated (or none at all), leading to a potentially poor coordination between the left and right ventricles. If too many cells are stimulated, selectivity and control of the pacing may suffer.

If a lead is incorrectly positioned, this may require a repositioning of the lead, which may be complicated and which one would like to avoid.

WO 02/087501 A2 describes a multi-electrode lead for a cardiac device, the lead having anywhere from three to 258 electrodes. The lead is preformed to a particular shape selected so that when implanted its electrodes are in contact with a particular cardiac tissue to sense cardiac activity and to provide CHF pacing.

US 9,008,768 B2 describes a catheter for capturing myocardium of a heart by delivering pacing signals to a location in the heart. The location is near an HIS bundle of the heart. The catheter has a proximal end for interfacing with an electrical pacing signal source and a distal end. The distal end includes a fixation mechanism that attaches the catheter to heart tissue. First and second electrodes are located at the distal end, each electrode being individually adjustable for providing pacing signals to the heart tissue.

### SUMMARY OF THE INVENTION

It is an object to provide a lead for a pacemaker device and a pacemaker device which in an easy and effective manner allow for a pacing at the HIS bundle.

In one aspect, a lead for a pacemaker device configured to provide for an intra-cardiac pacing at the HIS bundle comprises a shaft having a first end to be connected to a stimulation device of the pacemaker device, and a second end opposite to the first end, wherein the second end is to be implanted into intra-cardiac tissue in the vicinity of the HIS bundle of a patient's heart. An electrode arrangement is arranged on the shaft in proximity to the second end, the electrode arrangement comprising a multiplicity of electrodes to engage with intra-cardiac tissue in the vicinity of the HIS bundle.

By providing a pacing at the HIS bundle, advantages over a ventricular pacing may be achieved, for example allowing to avoid a reduction in the right ventricular filling volume, to improve the atrial to ventricular (A/V) synchrony, to avoid a predominant right ventricular pacing, and to improve the left ventricular (LV) to right ventricular (RV) synchrony. In particular, by pacing at the HIS bundle, the intrinsic conductive system of the heart is used, providing for a synchronous stimulation of the right ventricle and the left ventricle via the intrinsic right bundle branch and left bundle branch extending from the HIS bundle to extend about the right ventricle respectively the left ventricle. Hence, a synchronous pacing for both the right ventricle and the left ventricle is provided, avoiding an asynchronous and asymmetric pacing of the ventricles.

The lead is to be connected to a stimulation device and is to be implanted together with a stimulation device into a patient in order to extend from the stimulation device towards the patient's heart, in particular towards the right atrium in order to be placed on and engage with intra-cardiac tissue in the vicinity of the HIS bundle of the patient's heart, in particular collagen tissue of the HIS bundle. The second end herein is to be implanted into intra-cardiac tissue such that electrodes of the electrode arrangement arranged at the second end come to engage with the collagen tissue of the HIS bundle, such that by means of the electrodes of the electrode arrangement stimulation energy may be injected into the cells of the HIS bundle for providing for a pacing at the HIS bundle.

In one aspect, at least two of the multiplicity of electrodes of the electrode arrangement are arranged and formed on the shaft to simultaneously engage with the HIS bundle in an implanted state of the lead. The lead, hence, for implantation is inserted into intra-cardiac tissue in the vicinity of the HIS bundle such that the electrodes at the second end of the shaft together come to engage and contact with the cells of the HIS bundle, such that stimulation energy may be emitted from and may be injected towards the cells of the HIS bundle.

Electrodes of the electrode arrangement may for example axially be displaced with respect to each other along a longitudinal axis of extension of the shaft. In addition or alternatively, electrodes of the electrode arrangement may be circumferentially displaced with respect to one another. The distance between neighboring electrodes herein for example is less than 2 mm, beneficially less than 1 mm, less than 0.5 mm or even less than 0.25 mm. The electrodes are hence arranged close together and are spaced at a minimum distance with respect to each other.

Because the electrodes are arranged at the second end of the shaft of the lead across a confined area having a small axial extension (for example an axial extension of 2 to 3 mm), multiple electrodes of the electrode arrangement may simultaneously engage with collagen tissue of the HIS bundle in an implanted state of the lead. This may allow to selectively stimulate particular cells of the HIS bundle, in order to for example selectively stimulate the right bundle branch or the left bundle branch of the HIS bundle or portions thereof.

In one approach, one electrode from the multiplicity of electrodes of the electrode arrangement may be selected in order to inject stimulation energy, wherein the selection is such that the electrode which allows for a most efficient stimulation of the HIS bundle is used. This may be assessed for example in an initial threshold test in which electrodes are successively used for stimulation with varying amplitudes and an invoked cardiac activity is picked up and evaluated for example in an ECG measurement.

In another approach, a combination of multiple electrodes of the multiplicity of electrodes of the electrode arrangement may be selected in order to inject stimulation energy, wherein different combinations of electrodes may be used for stimulating different cells of the HIS bundle, for example for selectively stimulating cells of the right bundle branch and the left bundle branch. The stimulation of different cells herein may take place in a time-offset manner in order to for example compensate for pathological conduction delays in the HIS bundle and conduction pathways extending from the HIS bundle.

Because multiple electrodes are arranged on the second end of the shaft of the lead, a repositioning of the lead due to an incorrect placement of the lead in intra-cardiac tissue may be avoided. Because the electrodes are spatially displaced with respect to each other at the second end, one or multiple of the electrodes may be suitably selected for injecting a stimulus. Because the selection may be variably adapted dependent on the placement of the lead in intra-cardiac tissue, the requirements for accuracy for the placement of the lead are relieved, helping to reduce the need for a potential repositioning of the lead.

At least some electrodes for example may have the shape of ring electrodes circumferentially extending about the shaft at the second end, such ring electrodes being axially displaced with respect to each other.

In another aspect, at least one of the multiplicity of electrodes is formed by an electrically conductive patch extending, when viewed along a circumferential direction about the shaft, only across a portion of the shaft in proximity to the second end and being adjoined, when viewed along said circumferential direction, by a non-conductive section. Such an electrode hence does not extend circumferentially about the shaft at the second end to form a closed ring, but extends only across a circumferential portion of the shaft. The electrode hence does not provide for a symmetric emission of stimulation energy (when viewed in a plane perpendicular to the longitudinal axis of extension of the lead), but provides for an asymmetric injection of a stimulus. This may in addition improve selectivity for selectively stimulating cells of the HIS bundle.

Electrodes of the electrode arrangement may in principle be arranged in an arbitrary fashion on the shaft. For example, electrodes may be displaced with respect to each other both along the axial direction and along the circumferential direction, for example forming a regular matrix of electrodes at the second end of the shaft.

Conductive portions forming the electrodes may for example be made from an electrically conductive platinum material, whereas non-conductive portions may be formed for example from a ceramic material.

In one aspect, the lead may comprise a multiplicity of electrical conductors extending within an inner lumen of the shaft of the lead, each electrode of the multiplicity of electrodes being electrically connected to one of the electrical conductors. The electrical conductors herein may extend from the electrodes of the electrode arrangement at the second end towards an arrangement of contact elements at the first end of the lead, such that each electrode is connected, by means of a corresponding electrical conductor, to an associated contact element at the first end. Each contact element herein may be electrically fed by means of a stimulation device connected to the lead such that each electrode may be individually and selectively fed with electrical signals for injecting stimulation energy.

The lead may comprise, at the first end of the shaft, a plug connector to be connected to said stimulation device. By means of the plug connector, for example having the shape of an IS4 connector, a releasable connection in between the lead and the stimulation device may be established. Alternatively, the lead may be fixedly connected to the stimulation device for example by providing for a hardwiring of the electrical conductors to a circuitry of the stimulation device.

In one embodiment, the lead comprises a fixation device arranged in the region of the second end for fixing the lead to intra-cardiac tissue. The fixation device may for example have the shape of a screw arranged at the second end of the shaft, the screw protruding from the second end and allowing for a screw connection in between the lead and intra-cardiac tissue. In addition or alternatively, the fixation device may comprise wires for example in the shape of nitinol tines for engaging with intra-cardiac tissue.

In one embodiment, the fixation device is axially displaced from the second end towards the first end of the shaft, the electrodes of the electrode arrangement being placed on the shaft closer to the second end in comparison to the fixation device. In one embodiment, the fixation device may comprise tines which at one end are fixed to the shaft and at the other end are connected to a displacement element movably arranged on the shaft such that by moving the displacement element the tines may be deformed from a relaxed position in which they protrude radially from the shaft into a flat position in which they are approached towards the shaft, for example using an introducer sheath for receiving the tines during implantation of the lead. When removing the introducer sheath the tines of the fixation device may automatically revert towards their relaxed position and hence may be caused to radially protrude from the shaft of the lead in order to engage with intra-cardiac tissue such that a fixation of the lead on intra-cardiac tissue is achieved.

By axially displacing the fixation device from the electrode arrangement arranged on the second end of the shaft, the location of fixation of the lead on intra-cardiac tissue and the location of stimulation may be spatially separated from one another, allowing to achieve a secure fixation of the lead without risking a damaging of collagen tissue of the HIS bundle.

The fixation device, for example a screw or wires of the fixation device, may carry one or multiple electrodes of the electrode arrangement, such that the fixation device is electrically active for injecting stimulation energy. Alternatively, no electrodes are arranged on the fixation device such that the fixation device is electrically inactive.

In one embodiment, the lead comprises a wing arrangement arranged in proximity to the second end of the shaft, the wing arrangement having one or multiple wings configured to radially protrude from the shaft. Each wing of the wing arrangement may carry one or multiple electrodes of the electrode arrangement, electrodes being for example displaced along an axial direction of the shaft as well as along a transverse direction. By means of such wings the transverse extension of the electrode arrangement at the second end and hence in the vicinity of the HIS bundle in an implanted state of the lead may be increased, hence allowing to improve selectivity for the stimulation and increasing a potential stimulation area for efficiently injecting stimulation energy into the HIS bundle.

In one embodiment, the wings of the wing arrangement are flexibly deformable. Herein, for implanting the lead into intra-cardiac tissue the wings may assume a folded-in position in which the wings are approached towards a seating region of the shaft and hence are received closely on the shaft. In such a folded-in position the wings beneficially do not substantially protrude radially from the shaft at the second end. From the folded-in position the wings may be unrolled to assume a folded-out position in which the wings radially protrude from the shaft.

The folded-out position herein may correspond to a relaxed state of the wings. For implantation, the lead may be received within an introducer sheath, the introducer sheath holding the wings in their folded-in position in a strained fashion. When removing the introducer sheath, the wings automatically revert to their folded-out position such that they are caused to radially protrude from the shaft to engage with intra-cardiac tissue, in particular tissue of the HIS bundle for being able to efficiently inject stimulation energy into the HIS bundle.

In one embodiment, the wings may comprise one or multiple fixation members, for example in the shape of tines, which engage with intra-cardiac tissue in the folded-out position of the wings such that, by means of the fixation members, the wings are secured and fastened in their folded-out position.

The fixation members herein may be electrically active and may function as electrodes. Alternatively, the fixation members may be electrically inactive.

In one embodiment, the shaft, in the region of the second end, is configured to assume a curved shape in a relaxed state. The shaft hence is preformed such that it is curved at its second end. The shaft may for example tend to assume a spiral or helical shape when it is relaxed, wherein the shaft may be flexibly deformed from its curved shape, for example by inserting a mandrel into an inner lumen of the shaft, to allow implantation of the lead within a patient's heart.

In one aspect, a pacemaker device configured to provide for an intra-cardiac pacing comprises a stimulation device and at least one lead of the kind described above. The lead is to be implanted into a patient's heart, wherein the lead at its first end is connected to the stimulation device.

The stimulation device herein may be configured to provide for a selective multi-pole excitation. In particular, the stimulation device may comprise a multiplicity of connection elements connected to the at least one lead, in particular to contact elements of the electrical conductors associated with the electrodes of the electrode arrangement of the lead. A switching unit herein may serve to switch between the connection elements to selectively inject stimulation energy towards one or multiple of the electrodes of the electrode arrangement.

In particular, by means of the switching unit different pairs of electrodes may be energized for allowing a stimulation using different stimulation vectors (a vector being defined by a corresponding pair of electrodes). The stimulation device may comprise one or multiple pulse generators for generating stimulation pulses, wherein the stimulation pulses may be transmitted using different combinations of electrodes in a simultaneous or a time-offset manner for providing for a multipole HIS bundle pacing.

### BRIEF DESCRIPTION OF THE DRAWINGS

The various features and advantages of the present invention may be more readily understood with reference to the following detailed description and the embodiments shown in the drawings. Herein,
- Fig. 1: shows a schematic view of the human heart;
- Fig. 2: shows a view of an embodiment of a lead to be implanted into a patient's heart;
- Fig. 3: shows an enlarged view of a first end and a second end of a shaft of the lead;
- Fig. 4: shows a schematic view of the lead implanted into a patient's heart to engage, with the second end of the shaft, into intra-cardiac tissue to contact with cells of the HIS bundle;
- Fig. 5: shows a functional view of an embodiment of a stimulation device of the pacemaker device;
- Fig. 6: shows a view of another embodiment of a lead of a pacemaker device;
- Fig. 7: shows a view of yet another embodiment of a lead of a pacemaker device;
- Fig. 8: shows a view of yet another embodiment of a lead of a pacemaker device;
- Fig. 9: shows a view of yet another embodiment of a lead of a pacemaker device;
- Fig. 10: shows a view of an embodiment of an electrode on a second end of a shaft of a lead; and
- Fig. 11: shows a view of an embodiment of electrodes on a second end of a shaft of a lead.

### DETAILED DESCRIPTION

Subsequently, embodiments of the invention shall be described in detail with reference to the drawings. In the drawings, like reference numerals designate like structural elements.

It is to be noted that the embodiments are not limiting for the invention, but merely represent illustrative examples.

In the instant invention it is proposed to provide a pacemaker device comprising leads providing for an intra-cardiac pacing at the HIS bundle of a patient's heart.

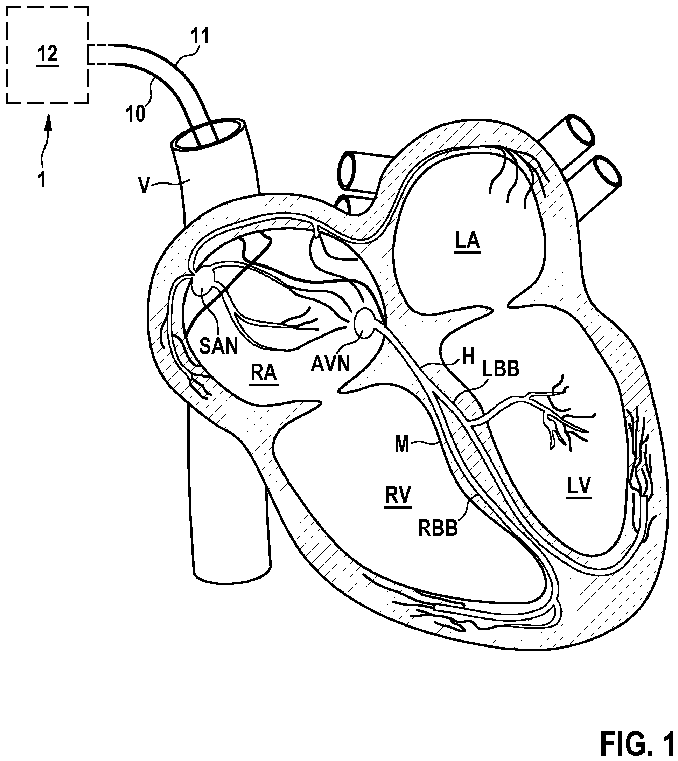

Fig. 1 shows, in a schematic drawing, the human heart comprising the right atrium RA, the right ventricle RV, the left atrium LA and the left ventricle LV, the so-called sinoatrial node SAN being located in the wall of the right atrium RA, the sinoatrial node SAN being formed by a group of cells having the ability to spontaneously produce an electrical impulse that travels through the heart's electrical conduction system, thus causing the heart to contract in order to pump blood through the heart. The atrioventricular node AVN serves to coordinate electrical conduction in between the atria and the ventricles and is located at the lower back section of the intra-atrial septum near the opening of the coronary sinus. From the atrioventricular node AVN the so-called HIS bundle H is extending, the HIS bundle H being comprised of heart muscle cells comprised of collagen tissue specialized for electrical conduction and forming part of the electrical conduction system for transmitting electrical impulses from the atrioventricular node AVN via the so-called right bundle branch RBB around the right ventricle RV and via the left bundle branch LBB around the left ventricle LV.

As electrical impulses from the atrioventricular node AVN are transmitted - in a healthy heart - collectively via the HIS bundle H towards the right bundle branch RBB and the left bundle branch LBB for stimulating the right ventricle RV and the left ventricle LV, HIS bundle pacing has the potential to synchronously pace the right ventricle RV and the left ventricle LV, hence avoiding a lack of synchrony in between the left ventricle RV and the right ventricle RV as it may occur for example in a right ventricular pacing.

For providing a stimulation at the HIS bundle H, a stimulation device 12 of a pacemaker device 1 uses leads 10, 11 extending from the (subcutaneously implanted) stimulation device 12 transvenously through the superior vena V into the right atrium RA to provide for an HIS bundle pacing at the HIS bundle H, as schematically indicated in Fig. 1. By means of such pacemaker device 1, stimulation energy is injected into the right atrial wall at the HIS bundle H in order to provide for a pacing at the HIS bundle H.

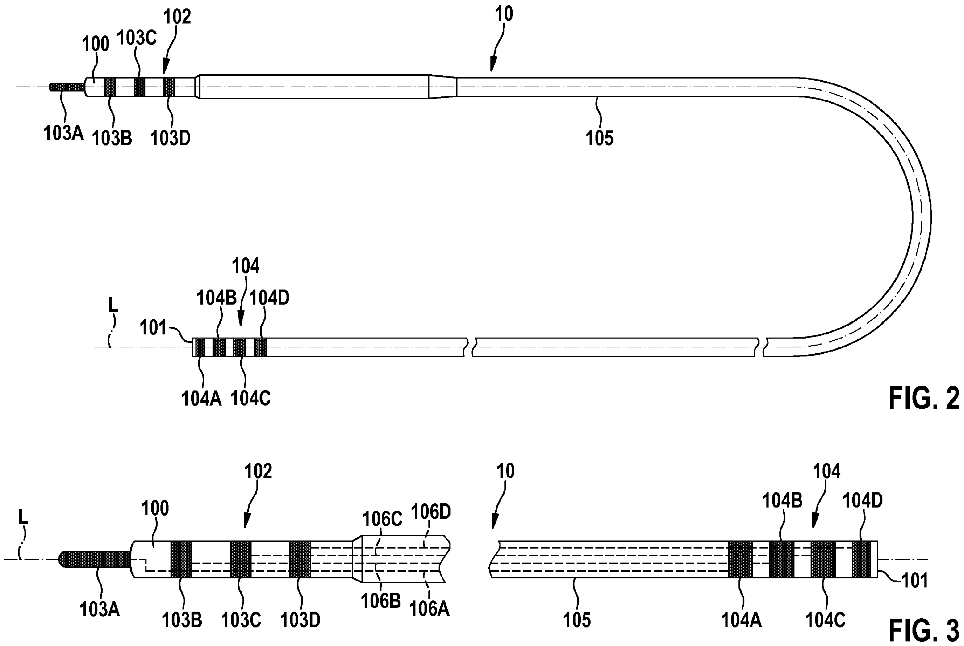

Fig. 2 shows an embodiment of a lead 10 of a pacemaker device 1, the lead 10 having a shaft 105 which, at a first end 100, is to be connected to a stimulation device 12 of the pacemaker device 1 and at a second end 101 is to be implanted into intra-cardiac tissue M in the vicinity of the HIS bundle H. The shaft 105 is flexibly deformable and has a generally longitudinal extension along a longitudinal axis L, as schematically indicated in Fig. 2.

As illustrated in Fig. 2 and in an enlarged view in Fig. 3, an electrode arrangement 104 comprising electrodes 104A-D is arranged on the second end 101 of the shaft 105. The electrodes 104A-D may for example have the shape of ring electrodes extending circumferentially about the shaft 105 at the second end 101 and being axially displaced with respect to each other along the longitudinal axis of extension L of the shaft 105.

As illustrated in Fig. 3, a multiplicity of electrical conductors 106A-D are received within an inner lumen of the shaft 105, each electrode 104A-D being electrically contacted to an associated electrical conductor 106A-D.

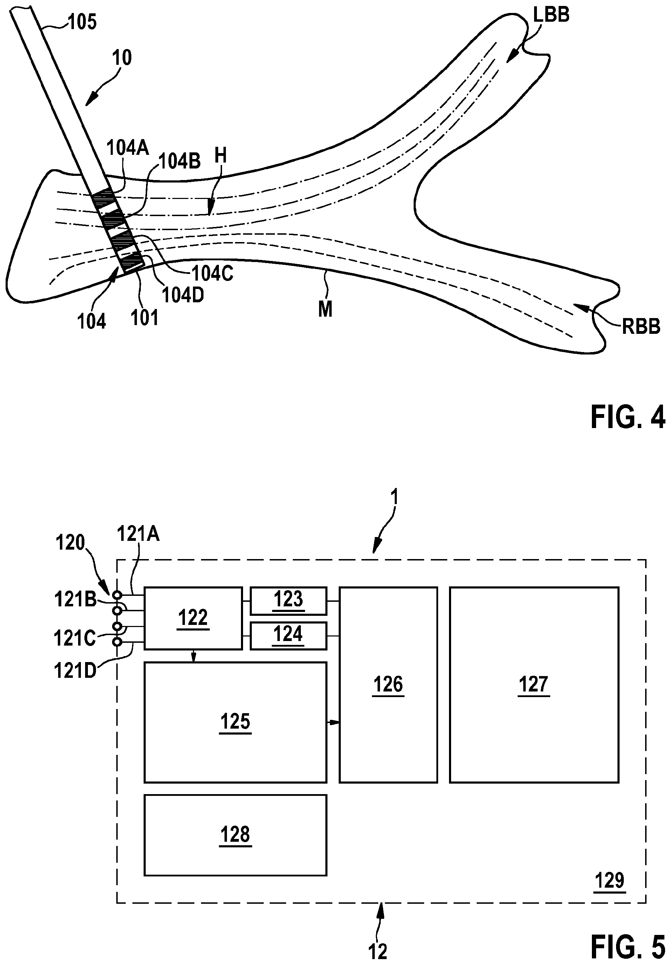

At the first end 100, the lead 10 comprises a plug connector 102 (for example an IS4 connector) for connection to the stimulation device 12. The plug connector 102 may in particular be inserted into a suitable connection opening with a socket connector 120 of the stimulation device 12, the plug connector 102 comprising contact elements 103A-D for electrically contacting with connection elements 121A-D of the stimulation device 12 (see Fig. 5).

Each of the contact elements 103A-D herein is electrically connected to an associated electrical conductor 106A-D, such that each contact element 103A-D is in electrical connection with a corresponding electrode 104A-D, allowing for an electrical feeding of the electrodes 104A-D via the contact elements 103A-D.

As schematically indicated in Fig. 4, the lead 10 is to be inserted by means of the second end 101 into intra-cardiac tissue M in the vicinity of the HIS bundle H. The electrodes 104A-D of the electrode arrangement 104 at the second end 101 of the shaft 105 herein are arranged within a comparatively small area of limited axial extension, neighboring electrodes 104A-D for example being spaced apart from one another by less than 2 mm, for example less than 1 mm, beneficially less than 0.5 mm or even 0.25 mm. In one embodiment, the axial dimension of the overall area in which the electrodes 104A-D are arranged on the second end 101 of the shaft 105 extends across an overall axial length of not more than 2 to 3 mm.

The arrangement and distribution of the electrodes 104A-D on the second end 101 of the shaft 105 is such that at least some of the electrodes 104A-D may together be brought into engagement with collagen tissue of the HIS bundle H, as schematically illustrated in Fig. 4.

Hence, the electrodes 104A-D come to rest within intra-cardiac tissue M, upon suitable implantation of the lead 10, such that the electrodes 104A-D collectively contact collagen tissue of the HIS bundle H and hence may be used to inject stimulation energy into the HIS bundle H for stimulating ventricular heart activity.

The lead 10 is connected to a stimulation device 12, as it is shown schematically in one embodiment in Fig. 5. The stimulation device 12 comprises a housing 129 for encapsulating electrical and electronic components. The stimulation device 12 is designed for a subcutaneous implantation, one or multiple leads 10, 11 extending from the stimulation device 12 for accessing a patient's heart for providing a stimulation and pacing of selected heart regions, in particular the left and right ventricles LA, RA.

The stimulation device 12 comprises connection elements 121A-D for electrically contacting contact elements 103A-D of a lead 10 connected to the stimulation device 12, the connection elements 121A-D for example extending towards a socket connector 120 for connection to the lead 10. The stimulation device 12 comprises a switching unit 122 connected to the connection elements 121A-D, the switching unit 122 being functionally connected to pulse generators 123, 124 for generating stimulation pulses for injection into intra-cardiac tissue M. The switching unit 122 serves to selectively switch between different electrodes 104A-D, in particular different pairs of electrodes 104A-D, for injecting a stimulation pulse by means of a selected pair of electrodes 104A-D, hence making use of a specific stimulation vector being defined by the arrangement of the particular pair of electrodes 104A-D on the second end 101 of the shaft 105.

By means of the pulse generators 123, 124 stimulation pulses may be injected using different pairs of electrodes in a simultaneous or time-offset manner during one or multiple heart cycles. The stimulation device 12 herein may comprise two or more pulse generators 123, 124, such that a multi-pole HIS pacing action may be obtained.

The pulse generators 123, 124 are connected to a timer unit 126 for defining a timing for the pulse generation. A detection unit 125 serves to receive signals via the electrodes 104A-D, the switching unit 122 being configured to switch between stimulation phases and detection phases such that, in a detection phase, intra-cardiac electrogram (IEGM) signals indicative of an atrial and/or ventricular activity may be received and evaluated by means of the stimulation device 12 for controlling a pacing action.

The stimulation device 12 comprises a control unit 127 for controlling operation of the stimulation device 12. An energy storage unit 128, in the shape of a battery, serves to supply electrical energy.

The stimulation device 12 may comprise further electrical or electronic components, for example a communication unit for communicating with an external control device outside of a patient, for example by telemetry. By means of such communication device diagnostic data may be transmitted towards the external device. In turn, the external device may transmit programming data, for example for adapting a stimulation routine, to the stimulation device 12.

By means of the electrode arrangement 104 comprising a multiplicity of spatially separated, selectively excitable electrodes 104A-D, an efficient injection of stimulation energy into the HIS bundle H may be achieved. In particular, in one approach one of the multiplicity of electrodes 104A-D or one combination of the multiplicity of electrodes 104A-D which is most suited as it best engages with tissue at the HIS bundle H may be used for stimulation. The one electrode 104A-D or the combination of electrodes 104A-D herein may be defined and selected for example by a detection threshold test in which, in an initial calibration phase, it is tested by taking an ECG measurement which electrode 104A-D or which combination of electrodes 104A-D may provide for a most efficient stimulation of the HIS bundle H.

In another approach, different combinations of electrodes 104A-D may selectively be used to inject stimulation energy in a simultaneous or time-offset manner in order to provide for a selective, multi-pole excitation of cells of the HIS bundle H, for example for providing a selective stimulation of the left bundle branch LBB and the right bundle branch RBB during one or multiple heart cycles. In this way for example differences in the conduction pathways of the left bundle branch LBB and the right bundle branch RBB may be compensated in order to provide for a synchronous, coordinated stimulation of the left and right ventricles by means of for example a time-offset injection of stimuli.

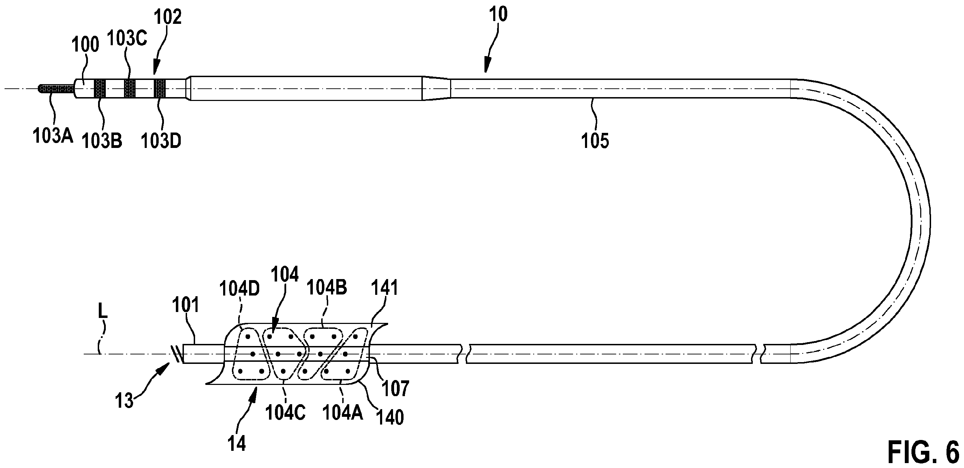

As illustrated in an embodiment in Fig. 6, the lead 10 may carry, at the second end 101, a fixation device 13, for example in the shape of a screw or in the shape of wires formed by nitenol tines to be engaged with intra-cardiac tissue M for fixing the lead 10 at the second end 101 of the shaft 105 to intra-cardiac tissue M. Such fixation device 13 may be present also on the lead 10 as schematically illustrated in Fig. 2.

In the particular embodiment of Fig. 6, a wing arrangement 14 comprising wings 140, 141 is arranged on the shaft 105, the wings 140, 141 radially protruding from the shaft 105 in a folded-out position, as illustrated in Fig. 6. The wings 140, 141 are flexibly deformable and may be rolled onto the shaft 105 to be received within a recessed seating region 107 on the shaft 105 in a folded-in state, such that the wings 140, 141 do not substantially protrude radially from the shaft 105, allowing for an implantation of the lead 10 within a patient's heart. For example, during implantation the lead 10, in the region of the wing arrangement 14, may be received within an introducer sheath, the wings 140, 141 of the wing arrangement 14 hence being deformed to assume the folded-in position in which they are approached towards the shaft 105. By removing the introducer sheath the wings 140, 141, due to their elastic tension, automatically reset to their folded-out position as illustrated in Fig. 6.

In the embodiment of Fig. 6, electrodes are arranged on the wings 140, 141, the electrodes forming for example particular groups of electrodes 104A-D associated with the contact elements 103A-D of the plug connector 102, the groups being selectively excitable for injecting stimulation energy into intra-cardiac tissue M.

As visible from Fig. 6, in the shown embodiment the electrodes may be displaced with respect to one another both axially and transversely to the longitudinal axis L of the shaft 105, the electrodes hence forming a distributed arrangement of excitation poles across the wings 140, 141.

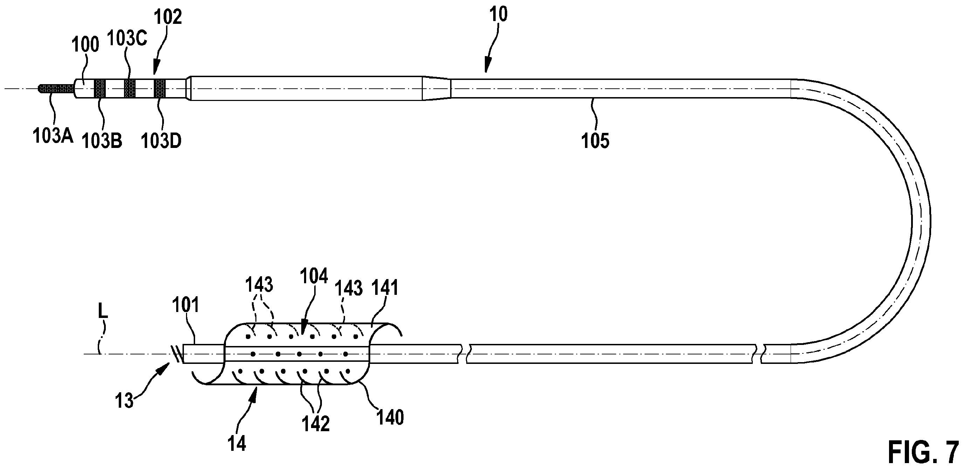

In a modified embodiment illustrated in Fig. 7, fixation members 142, 143 in the shape of wires formed by nitenol tines are arranged on the outer edges of the wings 140, 141, the fixation members 142, 143 serving to engage with intra-cardiac tissue M for fixing the wings 140, 141 in their folded-out position with respect to intra-cardiac tissue M.

Both the fixation device 13 and the fixation members 142, 143 of the wings 140, 141 may be electrically active or electrically inactive. In particular, the fixation device 13 may carry one or multiple electrodes forming part of the electrode arrangement 104. Alternatively or in addition, the fixation members 142, 143 may form electrodes of the electrode arrangement 104.

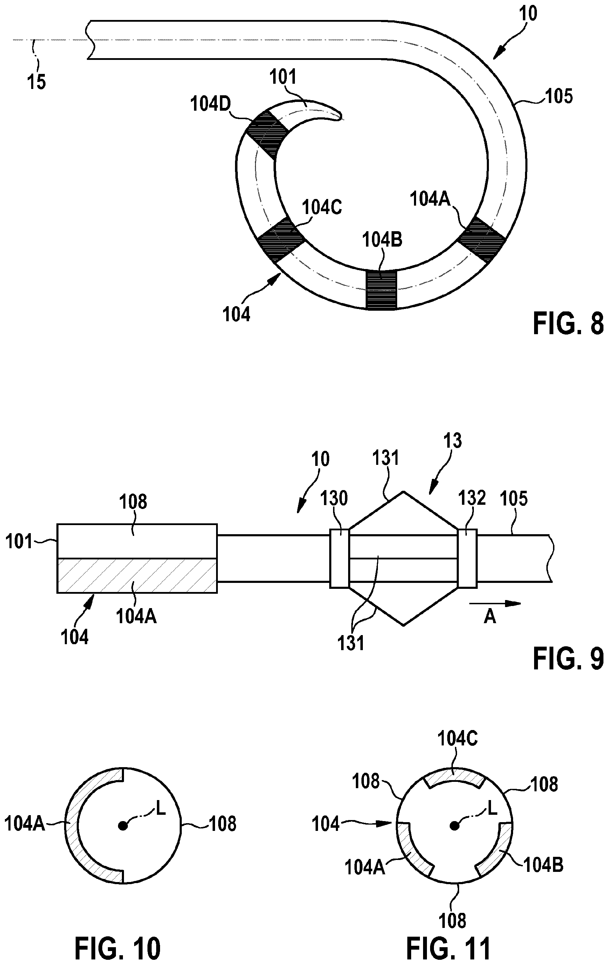

In an embodiment shown in Fig. 8, the shaft 105 of the lead 10 may, in the region of the second end 101, be preshaped to assume a curved shape when the shaft 105 is relaxed. In particular, the shaft 105 may be shaped to curve in a J-shape, a spiral shape or a helical shape, allowing the shaft 105 to engage with intra-cardiac tissue M for providing for a fixation of the lead 10 in intra-cardiac tissue M.

For implantation, a mandrel 15 may be inserted into an inner lumen of the shaft 105, the mandrel 15 serving to flexibly deform the shaft 105 from its relaxed, state, in particular to straighten the shaft 105 for guiding the lead 10 into the patient's heart during implantation.

A mandrel 15 may also be used for supporting a curving of the shaft 105 for implantation and engaging with intra-cardiac tissue M.

The electrodes 104A-D of the electrode arrangement 104 may, in particular in the embodiment of Fig. 2, have the shape of ring electrodes extending circumferentially about the shaft 105 at the second end 101. Alternatively, the electrodes 104A-D may extend - when viewed along a circumferential direction - only across a portion of the shaft 105, such that the electrodes 104A-D do not emit stimulation signals in a symmetric fashion about the longitudinal axis L, but in a directional, asymmetric fashion.

This is illustrated in one embodiment in Fig. 9, in which an electrode 104A is circumferentially adjoined by a non-conductive patch 108. As visible in Fig. 10, one electrode 104A may extend along a semicircle and hence may face towards one side of the lead 10 to emit stimulation energy predominantly towards that one side. Alternatively, multiple electrodes 104A-C may be spaced with respect to each other along a circumferential direction about the longitudinal axis L, as illustrated in an embodiment in Fig. 11, non-conductive, isolating patches being arranged in between neighboring electrodes 104A-C such that the electrodes 104A-C - when viewed circumferentially about the longitudinal axis L - are spatially and electrically separated from one another.

The lead 10, in the embodiment of Fig. 9, comprises a fixation device 13 having a multiplicity of tines 131 which, at one end, are fixedly connected to a fastening element 130 and, via the fastening element 130, to the shaft 105 such that the corresponding ends of the tines 131 are stationary on the shaft 105. The opposite, other ends of the tines 131 are connected to a moving element 132 movable along the shaft 105 in a sliding fashion along a moving direction A such that, by moving the moving element 132, the radial extension of the tines 131 from the shaft 105 can be controlled.

By moving the moving element 132 in the moving direction A with respect to the shaft 105 the tines 131 may be brought into a flat position in which they are approached towards the shaft 105 and do not substantially protrude radially from the shaft 105. The tines 131 herein may for example be received in an introducer sheath for implanting the lead 10 in a patient's heart. When removing the introducer sheath, the tines 131 automatically reset to their protruding, relaxed position as illustrated in Fig. 9, such that the tines 131 may engage with intra-cardiac tissue M for fixing the lead 10 on intra-cardiac tissue M.

In the embodiment of Fig. 9 the fixation device 13 is axially displaced with respect to the second end 101 of the lead 10 towards the first end 100. In particular, the fixation device 13 is arranged proximally from the electrode arrangement 104, such that the electrode arrangement 104 is spatially separated from the fixation device 13. This allows to anchor the lead 10 on intra-cardiac tissue M at a location removed from the HIS bundle H at which stimulation energy shall be injected, hence avoiding a risk of damaging collagen tissue of the HIS bundle H.

In the above described embodiments, electrodes 104A-D may for example be made from an electrically conductive platinum material.

The electrodes 104A-D may, in one embodiment, for example comprise a coating, in particular a fractal coating consisting of a material selected from iridium, tantalum or aluminum.

In one embodiment, a reservoir of a medical fluid, in particular a medication, may be arranged on the shaft 105 in the vicinity of the electrodes 104A-D of the electrode arrangement 104, the medication in particular having an anti-inflammatory effect.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

### LIST OF REFERENCE NUMERALS

- 1: Pacemaker device
- 10: Lead
- 100: Proximal end
- 101: Distal end
- 102: Connector
- 103A-D: Contact element
- 104: Electrode arrangement
- 104A-d: Electrode
- 105: Shaft
- 106A-D: Conductor
- 107: Seating region
- 108: Non-conductive portion
- 11: Lead
- 12: Stimulation device
- 120: Socket connector
- 121A-D: Connection element
- 122: Switching unit
- 123, 124: Pulse generator
- 125: Detection unit
- 126: Timing unit
- 127: Control unit
- 128: Energy storage unit
- 129: Housing
- 13: Fixation device
- 130: Fastening element
- 131: Tines
- 132: Moving element
- 14: Wing arrangement
- 140, 141: Wing
- 142, 143: Fixation members (tines)
- 15: Mandrel
- A: Moving direction
- H: HIS bundle
- L: Longitudinal axis
- LA: Left atrium
- LBB: Left bundle branch
- LV: Left ventricle
- M: Intra-cardiac tissue (myocardium)
- RA: Right atrium
- RBB: Right bundle branch
- RV: Right ventricle
- SAN: Sinoatrial node
- V: Superior vena

## Claims

1. A lead (10, 11) for a pacemaker device (1) configured to provide for an intra-cardiac pacing at the HIS bundle (H), the lead (10, 11) comprising:
a shaft (105) having a first end (100) to be connected to a stimulation device (12) of the pacemaker device (1), and a second end (101) opposite to the first end (100), wherein the second end (101) is to be implanted into intra-cardiac tissue (M) in the vicinity of the HIS bundle (H) of a patient's heart;
an electrode arrangement (104) arranged on the shaft (105) in proximity to the second end (101), the electrode arrangement (104) comprising a multiplicity of electrodes (104A-D) to engage with intra-cardiac tissue (M) in the vicinity of the HIS bundle (H).

2. The lead (10, 11) according to claim 1, wherein at least two of the multiplicity of electrodes (104A-D) are arranged and formed to simultaneously engage with the HIS bundle (H) in an implanted state of the lead (10, 11).

3. The lead (10, 11) according to claim 1 or 2, wherein at least two of the multiplicity of electrodes (104A-D) are displaced with respect to each other axially along a longitudinal axis (L) of extension of the shaft (105).

4. The lead (10, 11) according to claim 3, wherein the at least two of the multiplicity of electrodes (104A-D) are formed as ring electrodes circumferentially extending about the shaft (105) at the second end (101).

5. The lead (10, 11) according to one of the preceding claims, wherein at least one of the multiplicity of electrodes (104A-D) is formed by an electrically conductive patch extending, when viewed along a circumferential direction about the shaft (105), only across a portion of the shaft in proximity to the second end (101) and being adjoined, when viewed along said circumferential direction, by a non-conductive section (108).

6. The lead (10, 11) according to one of the preceding claims, comprising a multiplicity of electrical conductors (106A-D), each electrode of the multiplicity of electrodes (104A-D) being electrically connected to one of the electrical conductors (106A-D).

7. The lead (10, 11) according to claim 6, comprising a multiplicity of contact elements (103A-D) arranged in proximity to the first end (100) of the shaft (105), each contact element of the multiplicity of contact elements (103A-D) being electrically connected to one of the electrical conductors (106A-D).

8. The lead (10, 11) according to one of the preceding claims, comprising a plug connector (102) at the first end (100) to be connected to said stimulation device (12).

9. The lead (10, 11) according to one of the preceding claims, comprising a fixation device (13) arranged in the region of the second end (101) for fixing the lead (10, 11) to intra-cardiac tissue (M).

10. The lead (10, 11) according to one of the preceding claims, comprising a wing arrangement (14) arranged in proximity to the second end (101), the wing arrangement (14) having at least one wing (140, 141) configured to radially protrude from the shaft (105) and carrying at least one of the multiplicity of electrodes (104A-D).

11. The lead (10, 11) according to claim 10, wherein the at least one wing (140, 141) is flexibly deformable between a folded-in position in which the at least one wing (140, 141) is approached towards a seating region (107) of the shaft (105) and a folded-out position in which the at least one wing (140, 141) radially protrudes from the shaft (105).

12. The lead (10, 11) according to claim 11, wherein the at least one wing (140, 141) comprises at least one fixation member (142, 143) for fixing the at least one wing (140, 141) in the folded-out position with respect to intra-cardiac tissue (M).

13. The lead (10, 11) according to one of the preceding claims, wherein the shaft (105), in the region of the second end (102), is configured to assume a curved shape in a relaxed state and, for implantation, is flexibly deformable to assume another shape other than said curved shape.

14. A pacemaker device (1) configured to provide for an intra-cardiac pacing, the pacemaker device (1) comprising:
a stimulation device (12); and
at least one lead (10, 11) according to one of the preceding claims to be implanted into a patient's heart, wherein the at least one lead (10, 11), at its first end (100), is connected to the stimulation device (12).

15. The pacemaker device (1) of claim 14, wherein the stimulation device (12) comprises a multiplicity of connection elements (121A-D) connected to the at least one lead (10, 11) and a switching unit (122) for switching between the connection elements (121A-D) for transmitting stimulation signals to different electrodes of the multiplicity of electrodes (104A-D) of the at least one lead (10, 11).
